(19) Europäisches Patentamt

European Patent Office

Office européen des brevets

(11) Veröffentlichungsnummer: **0 249 167 B1**

# (12) EUROPÄISCHE PATENTSCHRIFT

(45) Veröffentlichungstag der Patentschrift: **29.09.93**

(21) Anmeldenummer: **87108170.9**

(22) Anmeldetag: **05.06.87**

(51) Int. Cl.⁵: **A61K 39/395**

(54) Verfahren zur Herstellung einer pasteurisierten Immunglobulinpraeparation.

(30) Priorität: **11.06.86 DE 3619565**

(43) Veröffentlichungstag der Anmeldung:
**16.12.87 Patentblatt 87/51**

(45) Bekanntmachung des Hinweises auf die
Patenterteilung:
**29.09.93 Patentblatt 93/39**

(84) Benannte Vertragsstaaten:
**AT BE CH DE ES FR GB GR IT LI LU NL SE**

(56) Entgegenhaltungen:
**EP-A- 0 058 993**
**EP-A- 0 124 506**
**EP-A- 0 139 975**
**EP-A- 0 177 836**
**EP-A- 0 196 761**

(73) Patentinhaber: **BEHRINGWERKE Aktiengesellschaft**
**Postfach 1140**
**D-35001 Marburg(DE)**

(72) Erfinder: **Müller, Hans**
**Lerchenstrasse 6**
**D-3563 Dautphetal(DE)**
Erfinder: **Geiger, Helmut, Dr.**
**Albert-Demnitz-Weg 5**
**D-3550 Marburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung einer Immunglobulin-Präparation, worin eine Lösung eines Immunglobulins zur Inaktivierung von vermehrungsfähigen Krankheitserregern in Gegenwart von Stabilisatoren erhitzt und gegebenenfalls gereinigt wird.

Es bestand ein Bedürfnis für ein Verfahren, welches durch Pasteurisierung eine Inaktivierung von Krankheitserreger wie Viren in Immunglobulinpräparationen unter Erhalt der Aktivität des Immunglobulins erlaubt. Bei der Herstellung von Immunglobulinen nach üblichen Verfahren wird ein potentielles Infektionsrisiko im allgemeinen vermindert. Der Gehalt an Viren oder viralen Antigenen wird häufig bis unter die Nachweisgrenze des gebrauchten Testsystems vermindert. Die Nachweisgrenze ist jedoch meist nicht ausreichend niedrig, um ein Infektionsrisiko eindeutig auszuschließen. Andererseits gibt es zum Nachweis von bestimmten Viruskontaminierungen keine entsprechenden Testsysteme. Eine Wärmebehandlung ist deshalb zweckmäßig.

Zur Vermeidung von Denaturierungen ist es notwendig, die Immunglobuline während der Wärmeeinwirkung zu stabilisieren. Zur sicheren Inaktivierung ist es unerläßlich, über einen längeren Zeitraum auf eine möglichst hohe Temperatur zu erwärmen.

In der EP-A 0 124 506 ist ein Verfahren beschrieben, bei dem zu einer Immunglobulinlösung Ammoniumsulfat gegeben und die Suspension über 10 Stunden auf 60°C erhitzt wird. Wenn jedoch eine Immunglobulinlösung nach Beispiel 16 jener Patentanmeldung behandelt wird, entsteht auch polymeres Immunglobulin. Die europäische Pharmakopoe lässt für ein Immunglobulin zur intramuskulären Anwendung nicht mehr als 10 % polymere Anteile zu.

In EP-A-0 144 714 ist beschrieben, daß eine Cohn-Fraktion II + III (J. Am. Chem. Soc. (1946) 68, 459) nur unter milden Bedingungen, vorzugsweise 52°C für etwa 30 Minuten, pasteurisiert werden kann, selbst wenn zuvor die Euglobuline entfernt und die Lösung durch Dialyse ethanolfrei gemacht wurde, und wobei trotzdem Aggregate entstehen. Es ist fraglich, ob eine sichere Virusinaktivierung unter diesen Bedingungen möglich ist.

In The Lancet, 19. November 1983, Seite 1198-99 ist ein Verfahren beschrieben, worin Human-Immunglobulin in Lösung mit 45 % (w:v) Sorbitol und 15 % (w:v) Glycin 10 h auf 60°C erhitzt wurde, und wobei weder ein Aktivitätsverlust noch ein Anstieg des Aggregatgehalts beobachtet wurde.

Ein Verfahren zur Pasteurisierung von Humanplasma unter Zusatz von Zuckeralkoholen, Aminosäuren oder Sacchariden ist in EP-A-0 139 975 beschrieben. Bei der Pasteurisierung von Plasma wird das Immunglobulin durch die anderen Plasmaproteine geschützt. Die stabilisierende Wirkung von Albumin auf IgG ist bekannt.

In der EP-A-0 196 761, die nach Art. 54(3) als Stand der Technik zu beachten ist, wird ein verfahren zur Virus-Inaktivierung in einer wässrigen Lösung eines gamma-Globulins beschrieben, wobei ein Monosaccharid, Disaccharid oder ein Zuckeralkohol zugegeben und die Lösung erhitzt wird.

Gegenstand der Erfindung ist ein verfahren zur Herstellung einer pasteurisierten Immunglobulinpräparation durch Erhitzen einer wässrigen Lösung eines Immunglobulins in Gegenwart eines Stabilisators, dadurch gekennzeichnet, daß eine Lösung des Immunglobulins in Gegenwart einer Carbonsäure oder eines ihrer Salze und/oder eines Saccharids unter Bedingungen erhitzt wird, daß vermehrungsfähige Krankheitserreger, besonders Hepatitisviren oder HTLV III ("Aids")-Viren inaktiviert werden.

Für diese Inaktivierung geeignete Bedingungen sind dem Fachmann bekannt. Üblich ist eine Erhitzung von etwa 10 Stunden auf etwa 60°C.

Diese Carbonsäure ist vorzugsweise eine aliphatische Carbonsäure, die vorzugsweise mit einer weiteren oder mehreren Carboxygruppen oder einer oder mehreren Aminogruppen oder einer oder mehreren Hydroxigruppen substituiert sein kann. Vorzugsweise enthält sie zwei bis zehn Kohlenstoffatome. Bevorzugt ist diese Carbonsäure Glycin, Glutaminsäure, Zitronensäure oder Weinsäure.

Vorzugsweise ist ein Salz einer Carbonsäure ein lösliches Metallsalz besonders ein Alkali-oder Erdalkalisalz, besonders das Natrium-oder Magnesiumsalz.

Als Salz der Glutaminsäure wird vorzugsweise ein Alkalisälz verwendet, besonders das Mononatriumsalz (Natriumglutamat).

Die Carbonsäuren oder ihre Salze können in einer Menge bis über die Sättigungsgrenze hinaus, vorzugsweise von 0,4 bis 0,6 g einem Milliliter der zu stabilisierenden Immunglobulinlösung zugesetzt werden.

Bevorzugt wird als Saccharid ein Mono-oder Disaccharid, besonders bevorzugt Saccharose verwendet.

Diese Saccharide werden vorzugsweise in einer Menge von 0,5 bis 1,0 g pro Milliliter der zu stabilisierenden Immunglobulinlösung zugegeben.

Die Carbonsäuren oder ihre Salze oder die Saccharide können in einer Menge bis über die Sättigungsgrenze hinaus der zu stabilisierenden Immunglobulinlösung zugesetzt werden.

Durch den Zusatz dieser Stoffe können die Immunglobuline ausfallen. Es wird in diesem Fall die entstandene Suspension erhitzt.

Der pH-Wert wird für die Erhitzung auf 5 - 8,5, bevorzugt 6,5 - 7,5 eingestellt.

Nach dem beanspruchten Verfahren kann man pasteurisierte Immunglobulinlösungen erhalten, deren Polymergehalt unter 10 % liegt. In den bevorzugten Ausführungen bleibt der Polymergehalt verglichen mit dem der unerhitzten Immunglobulinlösung innerhalb der methodischen Schwankungsbreite unverändert.

Das Ausgangsmaterial für das erfindungsgemäße Verfahren kann ein gereinigtes Immunglobulin sein, welches in der Literatur Gammaglobulin, IgG, Immunglobulin G oder in Anlehnung an J. Am. Chem. Soc. 71, 541 (1949) Fraktion II genannt wird. Solche Immunglobuline werden überwiegend aus immunglobulinhaltigen Fraktionen der Plasmafraktionierung hergestellt. Immunglobulinhaltige Fraktionen sind nach Vox. Sang. 7, 414 (1962) die Fraktion A oder nach J. Am. Chem. Soc. 68, 459 (1946) die Fraktion II und III.

Weiterhin können als Ausgangsmaterial modifizierte Immunglobuline eingesetzt werden. Solche Immunglobuline können durch chemische beispielsweise sulfitolytische Modifikation oder enzymatische Behandlung, beispielsweise peptische Abspaltung des Fc-Anteils, verändert sein. Die proteolytische Spaltung des Immunglobulin-Moleküls mit Pepsin bei pH 4 führt überwiegend zu $F(ab)_2$-Fragmenten mit einem Molekulargewicht von etwa 100 000 und einem in der analytischen Ultrazentrifuge bestimmten Sedimentationskoeffizienten von etwa 5 S (S = Svedberg-Einheit).

Solche Produkte enthalten neben ungespaltenem Immunglobulin von 7 S (Molekulargewicht etwa 150 000) praktisch keine Immunglobulin-Polymere. Es werden jedoch stärker fragmentierte Anteile mit einem Molekulargewicht kleiner als 5 S in Konzentration von unter 10 % beobachtet.

Überraschenderweise wurde gefunden, daß das beanspruchte Verfahren auch für Lösungen von Immunglobulinen geeignet ist, die Ethanol enthalten.

Bei der Gewinnung gereinigter Immunglobuline mittels Ethanol wird häufig als letzter Verfahrensschritt zur Konzentrierung eine Totalpräzipitation der Immunglobuline mit Ethanol vorgenommen und das Präzipitat durch Zentrifugation abgetrennt. Bei der Auflösung des Präzipitates resultieren Lösungen mit einem Restalkoholgehalt, welcher in der etwa 10 %igen Lösung etwa 4 Vol.-% beträgt. Die Entfernung des Ethanols erfolgt üblicherweise durch Gefriertrocknung oder Ultrafiltration. Würde die Erhitzung in der alkoholfreien z. B. ultrafiltrierten Lösung in Gegenwart von Stabilisatoren erfolgen, müßte nachfolgend eine erneute Ultrafiltration zur Entfernung dieser Zusätze erfolgen. Aus verfahrenstechnischen Gründen ist es daher vorteilhaft, die Erhitzung in Gegenwart von Ethanol durchzuführen.

Überraschend war gefunden worden, daß bei der Erhitzung von Immunglobulinen in Gegenwart von Ethanol bei 60°C über längere Zeiträume, z. B. 40 h, keine zunehmende Aggregation zu beobachten war, wenn eine Carbonsäure zugesetzt worden war.

Es ist bekannt, daß Ethanol bei höheren Temperaturen Immunglobuline denaturiert. Dementsprechend ergab eine Erhitzung von Immunglobulinen in Gegenwart von Carbonsäuren oder deren Salze als Stabilisatoren und Alkohol höhere Gehalte an Polymeren im Vergleich mit einer Arbeitsweise ohne Alkohol.

Eine Ethanol enthaltende Immunglobulinlösung ist beispielsweise eine gammaglobulin-haltige Fraktion, die nach Cohn et al., J. Am. Chem. Soc. (1946), 68, Seite 459 ff., Fraktion II + III oder nach Nitschmann (Kistner und Nitschmann, Vox Sang. (1962) 7, 414) Fraktion A genannt wird. Derartige Fraktionen enthalten neben Gammaglobulinen Lipoproteine, Euglobuline, Alpha-und Beta-Globuline und kleinere Mengen an Albumin. Der Gammaglobulin-Anteil liegt bei etwa 40-80 g auf 100 g Gesamtprotein. Bei der Auflösung von 100 g Fraktion II + III in 250 ml destilliertem Wasser beträgt der Alkoholgehalt der Lösung 4-5 ml/100 ml. Eine solche Fraktion II + III kann auf die erfindungsgemäße Weise pasteurisiert werden.

Tabelle 1 gibt die Gehalte an polymerem Immunglobulin nach Anwendung des beschriebenen Verfahrens im Vergleich zum Stand der Technik wieder. Es wurde jeweils 10 Stunden auf 60°C erhitzt. Der Immunglobulingehalt betrug 10-11 g Protein/100 ml Lösung. Der pH war 7.

## TABELLE 1

| Gehalte der Globulin-lösung in 100 ml | | | Zugabe zu 100 ml Globulinlösung | | | | Polymergehalt vor / nach Erhitzen* (g/100g Globulin) | |
|---|---|---|---|---|---|---|---|---|
| Ethanol (ml) | NaCl (g) | Glycin (g) | Stoff | Menge (g) | Stoff | Menge (g) | vor | nach |
| 0 | 0,3 | 2,25 | Ammonsulfat | 80 | - | - | 1,7 | 10,8 |
| 4 | 0,1 | 0 | Lysin, HCL | 100 | Glycin | 15 | 3,3 | 5,5 |
| 0 | 0,3 | 2,25 | Mononatrium-Citrat | 60 | - | - | 1,3 | 3,4 |
| 4 | 0,3 | 2,25 | "          " | 60 | - | - | 1,1 | 27,7 |
| 0 | 0,3 | 2,25 | Mono-Na-L-Glutamat | 60 | - | - | 1,1 | 1,4 |
| 4 | 0,3 | 2,25 | "    "   "  " | 60 | - | - | 1,1 | 4,5 |
| 2 | 0,3 | 2,25 | "    "   "  " | 60 | - | - | 1,2 | 1,8 |
| 2 | 0,12 | 0 | Glucose | 60 | - | - | 2,3 | 5 |
| 4 | 0,12 | 0 | " | 100 | Glycin | 15 | 3,0 | 3,1 |
| 2 | 0,12 | 0 | Fructose | 60 | - | - | 2,3 | 4,5 |
| 2 | 0,12 | 0 | Galactose | 60 | - | - | 2,3 | 5,1 |
| 4 | 0,1 | 0 | Saccharose | 100 | Glycin | 15 | 2,2 | 1,7 |
| 4 | 0,1 | 2,25 | " | 100 | Lysin, HCL | 15 | 3,3 | 4,1 |
| 4 | 0,1 | 0 | Mono-Na-L-Glutamat | 60 | Saccharose | 50 | 3,0 | 4,3 |
| 0 | 0,3 | 2,25 | Natriumtartrat | 50 | - | - | 1,3 | 3,6 |
| 4 | 0,3 | 2,25 | " | 50 | - | - | 1,1 | 17,3 |

\* Analytische Gelchromatografie mittels Ultrogel ACA 34. Die im Durchflußphotometer gemessenen Transmissionssignale werden in Extinktionen umgewandelt, ein flächiges Elutionsprofil erstellt und ausgewertet.

Die Tabelle zeigt, daß nach Anwendung des beanspruchten Verfahrens der unerwünschte Anstieg an Polymeren des Immunglobulins selbst in Anwesenheit von Alkohol in der Regel sehr niedrig ist. Dieser Befund wird auch durch andere Prüfverfahren bestätigt, zum Beispiel durch die Bestimmung der antikomplementären Aktivität.

Die Gehalte an bei der Erhitzung entstandener höhermolekularer Anteile können mittels bekannter Verfahren noch vermindert werden.

EP 0 249 167 B1

Dazu ist es vorteilhaft den zugesetzten Stabilisator, z. B. durch Ultrafiltration gegen ein für das gewählte Reinigungsverfahren geeignetes Ionenmilieu auszutauschen.

Die Erhitzungsdauer kann in gewissen Grenzen variiert werden.

Zur Prüfung der Effektivität des beschriebenen Verfahrens wurde eine Immunglobulinlösung mit einem Gehalt an Protein von 9,9 g/100 ml sowie 3,6 g/100 ml Ethanol mit 1 g Saccharose und 0,15 g Glycin auf 1 ml Lösung versetzt. Nach Zusatz von Rous-Sarkom-Virus (RSV) in einer Konzentration von $1 \times 10^4$ infektiösen RSV-Einheiten/ml (E/ml) wurde die Lösung auf 60°C erhitzt. Nach einstündiger Erhitzung war der Gehalt unter die Nachweisgrenze vermindert.

Tabelle 2 kann entommen werden, daß die beschriebene Erhitzung keinen Einfluß auf die Antikörperaktivität hat.

Tabelle 2

| Stabilisator | zugegebene Menge (g auf 1 ml) | Ethanol ml/100 ml | Erhitzung auf 60°C Stunden | Antikörpertiter | | |
| --- | --- | --- | --- | --- | --- | --- |
| | | | | Anti-Tetanus[1] | Anti-HBsAg[2] | Anti-Rubella[3] |
| – | – | – | – | 4048 | 0,22 | 30720 |
| Na-Glutamat | 0,6 | 2 | 10 | 4048 | 0,20 | 30720 |
| Glukose | 0,6 | 2 | 10 | 4048 | 0,19 | 30720 |
| – | – | – | – | 8096 | 0,31 | 30720 |
| Na-Glutamat | 0,6 | 4 | 10 | 8096 | 0,22 | 30720 |
| Na-Glutamat/Glycin | 0,6/0,15 | 4 | 10 | 8096 | 0,29 | 30720 |
| Na-Glutamat/Saccharose | 0,6/0,5 | 4 | 10 | 8096 | 0,28 | 30720 |
| Saccharose/Glycin | 1/0,15 | 4 | 10 | 8096 | 0,27 | 30720 |
| Glukose/Glycin | 1/0,15 | 4 | 10 | 8096 | 0,21 | 30720 |

1) Indirekter Haemagglutinationstest I.H.A. (reziproker Titer)
2) Radioimmunoassay (in Internationale Einheiten/ml)
3) Elisa (Enzyme linked immunosorbent assay; reziproker Titer)

Die folgenden Beispiele erläutern die Erfindung:

6

Beispiel 1:

Erhitzen einer Immunglobulinlösung mit Natriumglutamat

200 ml einer praktisch reinen Lösung von Immunoglobulin mit einem Eiweißgehalt von 90 g/l wurden unter Rühren mit 120 g Natriumglutamat (Mononatriumsalz der Glutaminsäure) versetzt. Dabei präzipitierte das Immunglobulin. Nach Einstellen des pH-Wertes auf 7 wurde die Suspension über 10 Stunden unter Rühren auf 60°C erhitzt. Nach Abkühlen auf Zimmertemperatur wurde das Präzipitat durch Filtration oder Zentrifugation abgetrennt. Der Niederschlag wurde in destilliertem Wasser aufgelöst. Das Glutamat wurde durch Dialyse oder Ultrafiltration entfernt. Die Lösung wurde auf den gewünschten Eiweißgehalt und isotonisch eingestellt.

Es wurden 110 ml mit einem Proteingehalt von 155 g/l erhalten. Der Gehalt an Polymeren betrug 1,6 % (unerhitzt 1,2 %).

Beispiel 2:

Erhitzen einer Immunglobulinlösung mit Saccharose und Glycin

Zu 89 l Immunglobulinlösung mit einem Kochsalzgehalt von 1 g/l und einem Eiweißgehalt von 97 g/l sowohl 3,6 Vol.-% Ethanol wurden 89 kg Saccharose sowie 13,3 kg Glycin gegeben. Nach Einstellung des pH-Wertes auf 7 wurde 10 Stunden unter Rühren auf 60°C erwärmt. Die Lösung wurde mit 100 l einer 0,3 g/100 ml Kochsalzlösung verdünnt und sterilfiltriert. Die Stabilisatoren wurden in bekannter Weise durch Ultrafiltration entfernt. Nachfolgend wurde die Lösung isotonisch und auf einen Eiweißgehalt von 160 g/l eingestellt.

Es wurden 51 l Lösung, mit einem Polymergehalt von 2,6 % (2 % in der unerhitzten Lösung) erhalten. Der Restgehalt an Saccharose beträgt 0,04 g/l.

Beispiel 3:

Zu 100 ml sulfoniertem Immunglobulin mit einem Eiweißgehalt von 100 g/l und einem Kochsalzgehalt von 3 g/l wurden 100 g Saccharose und 15 g Glycin gegeben. Nach Einstellung des pH-Wertes auf 7.3 wurde 10 Stunden unter Rühren auf 60°C erwärmt.

Anschließend wurde die Lösung auf Zimmertemperatur abgekühlt und mit 170 ml 0,3 g/100 ml Kochsalzlösung verdünnt. Die Stabilisatoren wurden durch Ultrafiltration entfernt. Der Austausch des Lösungsmittels geschah mit einer 0,3 g/100 ml Kochsalzlösung. Die Lösung des Immunglobulins wurde isotonisch und auf einen Eiweißgehalt von 50 g/l eingestellt.

Es wurden 195 ml Lösung mit einem Polymergehalt von 5,6 % (5,8 % in der unerhitzten Lösung) erhalten.

Beispiel 4:

13,7 l eines peptisch gespaltenen Immunglobulins mit einem Eiweißgehalt von 180 g/l und einem Kochsalzgehalt von 3,2 g/l wurden mit 13,5 l einer 0,3 g/100 ml Kochsalzlösung verdünnt. Die Erhitzung auf 60°C unter Rühren erfolgte nach Zugabe von 27,2 kg Saccharose und 4,08 kg Glycin bei pH 7. Anschließend wurde die Lösung auf Zimmertemperatur abgekühlt und mit 45 l einer 0,3 g/100 ml Kochsalzlösung verdünnt. Nach einer Sterilfiltration erfolgte die weitgehende Entfernung der zugesetzten Stabilisatoren mittels Ultrafiltration. Nachfolgend wurde die Lösung isotonisch und auf einen Eiweißgehalt von 50 g/l eingestellt. Es wurden 48 l Lösung mit einem restlichen Saccharosegehalt von 0,01 g/l erhalten. Der Gehalt an Komponenten bestimmter Molekulargewichte wurde in der analytischen Ultrazentrifuge wie folgt ermittelt:

```
Ausgangsmaterial:      S kleiner als 5  =    8,8 %
                       S etwa        5  =  75,5 %
                       S etwa        7  =  15,7 %
                       S größer als  7  =    0  %


Erhitztes Endprodukt: S kleiner als 5  =    8,1 %
                       S etwa        5  =  77,5 %
                       S etwa        7  =  14,4 %
                       S größer als  7  =    0  %
```

Beispiel 5:

Erhitzen einer gelösten ethanolhaltigen Fraktion II + III in Gegenwart von Ethanol

200 g Fraktion II + III wurden mit 500 ml destilliertem Wasser versetzt und unter Rühren gelöst. Der Lösung (etwa 700 ml) fügt man 700 g Saccharose und 0,3 bis 0,2 Mol/l Glycin zu. Nach Einstellung des pH-Wertes auf etwa 7 wurde unter Rühren über 10 Stunden bei 60 °C erhitzt. Die erhitzte Lösung wurde nachfolgend fraktioniert. Es wurden 304 ml Immunglobulinlösung mit einem Proteingehalt von 66 g/l erhalten. Der Polymergehalt betrug 1,1 %.

Eine unerhitzte Vergleichsaufarbeitung ergab, bei Verwendung von 200 g Fraktion II + III, 208 ml Immunglobulinlösung mit einem Proteingehalt von 96,8 g/l. Der Polymergehalt betrug 2,0 %.

**Patentansprüche**
**Patentansprüche für folgende Vertragsstaaten : BE, CH, DE, FR, GB, LI, NL, SE**

1.  Verfahren zur Herstellung einer pasteurisierten Immunglobulinpräparation durch Erhitzen einer wässrigen Lösung eines Immunglobulins in Gegenwart eines Stabilisators, dadurch gekennzeichnet, daß eine Lösung des Immunglobulins, die bis zu 5 % Ethanol enthält, in Gegenwart einer Carbonsäure oder eines ihrer Salze und/oder eines Saccharids unter Bedingungen erhitzt wird, daß vermehrungsfähige Krankheitserreger, besonders Hepatitisviren oder HTLV III ("Aids")-Viren inaktiviert werden.

2.  Verfahren nach Anspruch 1, dadurch gekennzeichnet, daß die Carbonsäure eine gegebenenfalls substituierte aliphatische Carbonsäure ist.

3.  Verfahren nach Anspruch 1, dadurch gekennzeichnet, daß die Carbonsäure eine aliphatische Carbonsäure mit zwei bis zehn Kohlenstoffatomen ist, die mit einer oder zwei weiteren Carboxygruppen, einer oder zwei Aminogruppen und/oder mit einer oder mehreren Hydroxigruppen substituiert ist.

4.  Verfahren nach Anspruch 1, dadurch gekennzeichnet, daß die Carbonsäure Glycin, Glutaminsäure, Zitronensäure oder Weinsäure ist.

5.  Verfahren nach Anspruch 1, dadurch gekennzeichnet, daß das Saccharid ein Mono- oder Disaccharid ist.

6.  Verfahren nach Anspruch 1, dadurch gekennzeichnet, daß das Saccharid Glucose, Fructose, Galactose oder Saccharose ist.

**Patentansprüche für folgende Vertragsstaaten : AT, ES, GR, IT, LU**

1. Verfahren zur Herstellung einer pasteurisierten Immunglobulinpräparation durch Erhitzen einer wässrigen Lösung eines Immunoglobulins in Gegenwart eines Stabilisators, dadurch gekennzeichnet, daß eine Lösung eines Immunglobulins in Gegenwart einer Carbonsäure oder eines ihrer Salze und/oder eines Saccharids unter Bedingungen erhitzt wird, daß vermehrungsfähige Krankheitserreger, besonders Hepatitisviren oder HTLV III("Aids")-Viren inaktiviert werden.

2. Verfahren nach Anspruch 1, dadurch gekennzeichnet, daß die Carbonsäure eine gegebenenfalls substituierte aliphatische Carbonsäure ist.

3. Verfahren nach Anspruch 1, dadurch gekennzeichnet, daß die Carbonsäure eine aliphatische Carbonsäure mit zwei bis zehn Kohlenstoffatomen ist, die mit einer oder zwei weiteren Carboxygruppen, einer oder zwei Aminogruppen und/oder mit einer oder mehreren Hydroxigruppen substituiert ist.

4. Verfahren nach Anspruch 1, dadurch gekennzeichnet, daß die Carbonsäure Glycin, Glutaminsäure, Zitronensäure oder Weinsäure ist.

5. Verfahren nach Anspruch 1, dadurch gekennzeichnet, daß das Saccharid ein Mono- oder Disaccharid ist.

6. Verfahren nach Anspruch 1, dadurch gekennzeichnet, daß das Saccharid Glucose, Fructose, Galactose oder Saccharose ist.

**Claims**
**Claims for the following Contracting States : BE, CH, DE, FR, GB, LI, NL, SE**

1. A process for the preparation of a pasteurized immunoglobulin preparation by heating an aqueous solution of an immunoglobulin in the presence of a stabilizer, which comprises heating a solution of the immunoglobulin, which contains up to 5% ethanol, in the presence of a carboxylic acid or one of its salts and/or of a saccharide under conditions such that pathogens which are capable of reproduction, in particular hepatitis viruses or HTLV III ("Aids") viruses, are inactivated.

2. The process as claimed in claim 1, wherein the carboxylic acid is an optionally substituted aliphatic carboxylic acid.

3. The process as claimed in claim 1, wherein the carboxylic acid is an aliphatic carboxylic acid which has two to ten carbon atoms and is substituted with one or two additional carboxyl groups, one or two amino groups and/or one or more hydroxyl groups.

4. The process as claimed in claim 1, wherein the carboxylic acid is glycine, glutamic acid, citric acid or tartaric acid.

5. The process as claimed in claim 1, wherein the saccharide is a mono- or disaccharide.

6. The process as claimed in claim 1, wherein the saccharide is glucose, fructose, galactose or sucrose.

**Claims for the following Contracting States : AT, ES, GR, IT, LU**

1. A process for the preparation of a pasteurized immunoglobulin preparation by heating an aqueous solution of an immunoglobulin in the presence of a stabilizer, which comprises heating a solution of an immunoglobulin in the presence of a carboxylic acid or one of its salts and/or of a saccharide under conditions such that pathogens which are capable of reproduction, in particular hepatitis viruses or HTLV III ("Aids") viruses, are inactivated.

2. The process as claimed in claim 1, wherein the carboxylic acid is an optionally substituted aliphatic carboxylic acid.

3. The process as claimed in claim 1, wherein the carboxylic acid is an aliphatic carboxylic acid which has two to ten carbon atoms and is substituted with one or two additional carboxyl groups, one or two amino groups and/or one or more hydroxyl groups.

4. The process as claimed in claim 1, wherein the carboxylic acid is glycine, glutamic acid, citric acid or tartaric acid.

5. The process as claimed in claim 1, wherein the saccharide is a mono- or disaccharide.

6. The process as claimed in claim 1, wherein the saccharide is glucose, fructose, galactose or sucrose.

**Revendications**
**Revendications pour les Etats contractants suivants : BE, CH, DE, FR, GB, LI, NL, SE**

1. Procédé de préparation d'une composition d'immunoglobuline pasteurisée, par chauffage d'une solution aqueuse d'une immunoglobuline en présence d'un stabilisant, caractérisé en ce qu'on chauffe une solution de l'immunoglobuline, qui contient jusqu'à 5 % d'éthanol, en présence d'un acide carboxylique ou d'un de ses sels et/ou d'un saccharide, dans des conditions telles que les germes pathogènes capables de se multiplier, en particulier les virus de l'hépatite ou les virus HTLV III (virus du SIDA) sont inactivés.

2. Procédé selon la revendication 1, caractérisé en ce que l'acide carboxylique est un acide carboxylique aliphatique éventuellement substitué.

3. Procédé selon la revendication 1, caractérisé en ce que l'acide carboxylique est un acide carboxylique aliphatique comportant deux à dix atomes de carbone, qui est substitué par un autre (ou deux autres) groupe(s) carboxyle, par un ou deux groupe(s) amino et/ou par un ou plusieurs groupe(s) hydroxyle.

4. Procédé selon la revendication 1, caractérisé en ce que l'acide carboxylique est la glycine, l'acide glutamique, l'acide citrique ou l'acide tartrique.

5. Procédé selon la revendication 1, caractérisé en ce que le saccharide est un mono- ou disaccharide.

6. Procédé selon la revendication 1, caractérisé en ce que le saccharide est le glucose, le fructose, le galactose ou le saccharose.

**Revendications pour les Etats contractants suivants : AT, ES, GR, IT, LU**

1. Procédé de préparation d'une composition d'immunoglobuline pasteurisée, par chauffage d'une solution aqueuse d'une immunoglobuline en présence d'un stabilisant, caractérisé en ce qu'on chauffe une solution d'une immunoglobuline en présence d'un acide carboxylique ou d'un de ses sels et/ou d'un saccharide, dans des conditions telles que les germes pathogènes capables de se multiplier, en particulier les virus de l'hépatite ou les virus HTLV III (virus du SIDA) sont inactivés.

2. Procédé selon la revendication 1, caractérisé en ce que l'acide carboxylique est un acide carboxylique aliphatique éventuellement substitué.

3. Procédé selon la revendication 1, caractérisé en ce que l'acide carboxylique est un acide carboxylique aliphatique comportant deux à dix atomes de carbone, qui est substitué par un autre (ou deux autres) groupe(s) carboxyle, par un ou deux groupe(s) amino ou par un ou plusieurs groupe(s) hydroxyle.

4. Procédé selon la revendication 1, caractérisé en ce que l'acide carboxylique est la glycine, l'acide glutamique, l'acide citrique ou l'acide tartrique.

5. Procédé selon la revendication 1, caractérisé en ce que le saccharide est un mono- ou disaccharide.

6. Procédé selon la revendication 1, caractérisé en ce que le saccharide est le glucose, le fructose, le galactose ou le saccharose.